# FASCICULE DE BREVET EUROPEEN

(11) **EP 2 483 429 B1**
(45) Date de publication et mention de la délivrance du brevet: **16.12.2015**
(21) Numéro de dépôt: 10776777.4
(22) Date de dépôt: 01.10.2010
(51) Int. Cl.: C12R 1/41, C08J 11/10

(54) **SOUCHES BACTERIENNES ET LEURS VARIANTS CAPABLES DE DÉGRADER L'ACIDE POLYLACTIQUE ET LEURS UTILISATIONS**
BAKTERIEN-STÄMME UND VARIANTEN MIT DER FÄHIGKEIT POLY-MILCHSÄURE ABZUBAUEN UND DEREN VERWENDUNGEN
BACTERIAL STRAINS AND VARIANTS CAPABLE OF DEGRADING POLY (LACTIC ACID) AND THEIR USES

(30) Priorité: 02.10.2009 FR 0956898
(43) Date de publication de la demande: 08.08.2012
(73) Titulaire: Centre National de la Recherche Scientifique (CNRS), 75016 Paris (FR); Universite De Poitiers, 86000 Poitiers (FR)
(72) Inventeur: FERREIRA, Thierry, F-86240 Iteuil (FR); AUCHER, Willy, F-75015 Paris (FR)
(74) Mandataire: Cabinet Plasseraud
(86) Numéro de dépôt international: PCT/FR2010/052076
(87) Numéro de publication internationale: WO 2011/039489

(56) Documents cités:
- JP-A- 2007 000 109
- MATSUDA EMIKO ET AL: "Gene cloning and molecular characterization of an extracellular poly(L-lactic acid) depolymerase from Amycolatopsis sp strain K104-1", JOURNAL OF BACTERIOLOGY, vol. 187, no. 21, novembre 2005 (2005-11), pages 7333-7340, XP002616534, ISSN: 0021-9193
- AKUTSU-SHIGENO YUKIE ET AL: "Cloning and sequencing of a poly(DL-lactic acid) depolymerase gene from Paenibacillus amylolyticus strain TB-13 and its functional expression in Escherichia coli.", APPLIED AND ENVIRONMENTAL MICROBIOLOGY, vol. 69, no. 5, mai 2003 (2003-05), pages 2498-2504, XP002616535, ISSN: 0099-2240
- SAKAI KENJI ET AL: "Isolation of a thermophilic poly-L-lactide degrading bacterium from compost and its enzymatic characterization", JOURNAL OF BIOSCIENCE AND BIOENGINEERING, vol. 92, no. 3, 2001, pages 298-300, XP002616536, ISSN: 1389-1723
- SHIMAO M: "BIODEGRADATION OF PLASTICS", 1 janvier 2001 (2001-01-01), CURRENT OPINION IN BIOTECHNOLOGY, LONDON, GB LNKD- DOI:10.1016/S0958-1669(00)00206-8, PAGE(S) 242 - 247, XP001029668, ISSN: 0958-1669 page 243, colonne de gauche, alinéa 2
- ZUO Y ET AL: "Isolation of the exoelectrogenic bacterium Ochrobactrum anthropi YZ-1 by using a U-tube microbial fuel cell", APPLIED AND ENVIRONMENTAL MICROBIOLOGY 200805 US LNKD- DOI:10.1128/AEM.02732-07, vol. 74, no. 10, mai 2008 (2008-05), pages 3130-3137, XP002583543, ISSN: 0099-2240
- THOMA B ET AL: "Identification and antimicrobial susceptibilities of Ochrobactrum spp", INTERNATIONAL JOURNAL OF MEDICAL MICROBIOLOGY, URBAN UND FISCHER, DE, vol. 299, no. 3, 1 March 2009 (2009-03-01) , pages 209-220, XP025995606, ISSN: 1438-4221, DOI: 10.1016/J.IJMM.2008.06.009 [retrieved on 2008-08-27]

### SEQUENCE LISTING

<110> CENTRE NATIONAL DE LA RECHERCHE SCIENTIFIQUE (CNRS) UNIVERSITE DE POITIERS
<120> SOUCHES BACTERIENNES ET LEURS VARIANTS CAPABLES DE DÉGRADER L'ACIDE POLYLACTIQUE ET LEURS UTILISATIONS
<130> BCT100331QT
<160> 2
<170> PatentIn version 3.5
<210> 1
   <211> 57
   <212> DNA
   <213> Artificial
<220>
   <223> primer sequence
<400> 1
   cgcccgccgc gccccgcgcc cgtcccgccg cccccgcccg cctacgggag gcagcag 57
<210> 2
   <211> 20
   <212> DNA
   <213> Artificial
<220>
   <223> primer sequence
<400> 2
   ccgtcaattc ctttragttt 20

## Description

### DOMAINE DE L'INVENTION

L'invention concerne des souches bactériennes et les variants de ces souches bactériennes capables de dégrader l'acide polylactique et leurs utilisations.

### INTRODUCTION

Les matériaux polymères, appelés plus communément « plastiques », sont rencontrés dans de nombreux domaines d'application du fait de leurs propriétés physico-chimiques polyvalentes et de leur faible coût de production. Cependant, leur utilisation intensive au cours des dernières décennies, couplée à leur résistance à la dégradation, pose aujourd'hui un problème majeur dans le traitement des rejets qui leur sont associés : à l'horizon 2010, les prévisions estiment que la consommation mondiale de plastiques sera comprise entre 180 et 258 millions de tonnes.

Malgré des efforts importants dans la gestion des déchets, comme le développement des circuits de recyclage, le traitement des plastiques pose encore de nombreux problèmes et l'incinération ou la mise en décharge restent souvent les seules alternatives à leur élimination définitive.

Depuis quelques années, une nouvelle génération de matériaux est apparue sur le marché : les matériaux plastiques « biodégradables » (écopolymères ou encore bioplastiques). Il s'agit d'une gamme de matériaux bien particulière, dont la formulation est adaptée à leur prise en charge et à leur dégradation par les micro-organismes de l'environnement. Malheureusement, les écopolymères actuellement disponibles sur le marché ne sont dégradables que dans des conditions extrêmement favorables qui ne sont généralement pas rencontrées en milieu naturel.

Parmi les écopolymères, l'acide polylactique (PLA) est l'un des plus prometteurs : d'origine naturelle, il est depuis quelques années compétitif d'un point de vue commercial, du fait de la mise en place de production à grande échelle. De plus, ses qualités physico-chimiques peuvent permettre de répondre à différentes applications. Le PLA est considéré à la fois comme biodégradable et biocompatible, ce qui permet en particulier son utilisation pour l'élaboration de matériaux en contact avec des tissus vivants (i. e. pour des utilisations biomédicales comme les implants, les sutures, l'encapsidation de molécules d'intérêt thérapeutique...). La synthèse du PLA est un processus en plusieurs étapes qui débute par la production d'acide lactique, et se termine par la polymérisation des monomères. Comme les monomères de base (l'acide lactique) sont obtenus par fermentation à partir de ressources renouvelables (carbohydrates), le PLA est considéré à travers le monde comme un matériau d'avenir dans le contexte d'un "développement durable". En outre, sa consommation en 2006 était de l'ordre de 60000 tonnes par an, alors qu'uniquement 30 % de l'acide lactique disponible industriellement était utilisé pour sa production. Ce polymère possède donc un important potentiel de développement.

Néanmoins, malgré l'ensemble de ces qualités, la stabilité des liaisons esters entre les monomères d'acide lactique constituant le PLA a pour conséquence de diminuer la dégradabilité de ce matériau. En effet, le PLA n'est totalement dégradable qu'en conditions de compost industriel à des températures de 60°C ou supérieures. Durant ce processus, la dégradation des polymères de PLA est la combinaison d'une dégradation abiotique (par simple hydrolyse des liaisons esters entre les monomères, qui est accélérée à chaud) et, dans un second temps, d'une biodégradation par les micro-organismes du compost, qui hydrolysent les oligomères résiduels jusqu'à l'étape de minéralisation finale (l'ensemble constituant une dégradation dite "biotique"). L'utilisation de telles températures étant coûteuse d'un point de vue énergétique, des alternatives classiques pour le traitement de ces déchets restent l'incinération ou le dépôt en décharge, qui sont particulièrement délétères d'un point de vue environnemental.

L'utilisation de micro-organismes ou d'enzymes purifiées pour améliorer la biodégradation du PLA a été envisagée. Des travaux rapportent la dégradation d'oligomères (poids moléculaire de l'ordre de 1000 Da) par *Fusarium moniliforme* et *Penicillium roqueforti* (Torres, A. et al. 1996, Screening of microorganisms for biodegradation of poly(lactic-acid) and lactic acid-containing polymers, vol. 62, 2393-2397), par des actinomycètes comme *Amycolatopsis sp.* (Pranamuda, H. et al., 1997 Polylactide Degradation by an Amycolatopsis sp, vol. 63, 1637-1640), *Saccharothrix waywayandensis* (Jarerat, A., and Tokiwa, Y., 2003, Biotechnology Letters 25, 401-404), *Kibdelosporangium aridum* (Jarerat, A. et al. 2003, Biotechnology Letters 25, 2035-2038) ou par des bactéries comme *Bacillus brevis* (Tomita, K., 1999, Journal of Bioscience and Bioengineering 87, 752-755) ou encore *Paenibacillus amylolyticus* (Teeraphatpornchai, T. et al., 2003, Biotechnology Letters 25, 23-28). D'autres travaux décrivent la dégradation du PLA par des microorganismes appartenant au genre *Staplylococcus* ou au genre *Streptomyces* (US 5,925,556 et US 6,066,492). De plus, la dégradation de PLA de faible masse moléculaire (de l'ordre de 2000 Da) par une enzyme de type estérase telle que la lipase de *Rhizopus delemer* a été démontrée (Fukuzaki, H., 1989, European Polymer Journal 25, 1019-1026). L'enzyme de type cutinase de la levure *Cryptococcus sp.* S2 est également capable d'hydrolyser le PLA (Masaki, K., et al., 2005, Cutinase-Like Enzyme from the Yeast Cryptococcus sp. Strain S-2 Hydrolyzes Polylactic Acid and Other Biodegradable Plastics 71, 7548-7550). Enfin, des études ont montré que certaines lipases commerciales, et en particulier la lipase PL de *Alcaligenes sp*., permettaient la dégradation totale du PLA en 20 jours, toutefois dans des conditions de température et pH particulières (55°C, pH 8,5) (Hoshino, A., and Isono, Y. (2002). Degradation of aliphatic polyester films by commercially available lipases with special reference to rapid and complete degradation of poly(L-lactide) film by lipase PL derived from Alcaligenes sp. Biodegradation *13*, 141-147). De la même manière, des protéases commerciales ont été testées pour cette capacité. Certaines, en particulier les protéases alcalines issues du genre *Bacillus* et notamment la Savinase 16.0L, se sont révélées efficaces. Toutefois, aucune de ces enzymes n'est capable de dégrader des films de PLA industriels (les films expérimentaux ayant une surface amorphe, ils sont plus susceptibles à la dégradation que ceux de l'industrie dont la structure est plus cristalline) (Oda, Y. et al., 2000, Degradation of Polylactide by Commercial Proteases. Journal of Polymers and the Environment 8, 29-32). *In fine*, l'enzyme commerciale la plus couramment utilisée pour étudier la biodégradation du PLA est la protéinase K de *Tritirachium album* (Oda, Y. et al., 2000, Degradation of Polylactide by Commercial Proteases. Journal of Polymers and the Environment 8, 29-32). Emiko et al. décrivent une souche spécifique *Amycolatpsis* susceptible de dégrader l'acide polylactique (Emiko et al. journal of Bacteriology, 2005). De même, Akutus-Shigeno et al. décrivent des souches particulières *Paenibacillus amylolyticus* qui expriment une dépolymérase susceptible de dégrader l'acide polylactique (Akutsu-Shigeno et al., Applied and Environmental Microbiology, 2003). Enfin, Sakal et al. décrivent une souche particulière de *Bacillus smithii* dont le milieu de culture est susceptible de dégrader l'acide polylactique (Sakal et al., Journal of Bioscience and Bioengineering, 2001).

Plusieurs limitations sont néanmoins associées à ces procédés, et aucun d'eux n'a encore fait l'objet d'un développement industriel. Tout d'abord, dans de nombreux cas, une dégradation significative du PLA n'est observée qu'avec des enzymes purifiées, ce qui implique un coût de production très élevé, généralement incompatible pour des utilisations d'usage courant, à valeur ajoutée relativement modérée, comme les pots horticoles, par exemple. Le second point est que les micro-organismes utilisés sont pour la plupart, soit des souches de laboratoire, soit des souches isolées d'environnements non-conventionnels. Ceci a pour conséquence que la plupart des activités enzymatiques décrites sont en conditions sub-optimales dans le milieu naturel. Enfin, la plupart des micro-organismes et enzymes testés ne sont capables d'hydrolyser que des oligomères de faible poids moléculaire, ce qui est une limite importante pour leur utilisation dans des processus industriels qui utilisent des polymères de grande taille.

Il existe donc un réel besoin pour de nouveaux moyens de dégradation du PLA.

### RÉSUMÉ DE L'INVENTION

Les inventeurs ont eu le mérite d'isoler une souche de bactérie appartenant au genre *Ochrobactrum* présentant des propriétés surprenantes de dégradation de l'acide polylactique.

Il est décrit une souche de bactérie du genre *Ochrobactrum*, caractérisée en ce que ladite souche est capable de dégrader l'acide polylactique.

L'invention a pour objet une souche de bactérie du genre *Ochrobactrum* déposée selon la Traité de Budapest le 23 juillet 2009 au nom du Centre National de la Recherche Scientifique à la Collection Nationale de Cultures de Microorganismes sous le numéro CNCM I-4212, ou un variant de ladite souche, ledit variant étant capable de dégrader l'acide polylactique et ledit variant étant un variant naturel de ladite souche ou un variant de ladite souche comprenant au moins une mutation dans son génome.

L'invention concerne aussi un mélange de microorganismes caractérisé en ce qu'il comprend une souche ou un variant de ladite souche selon l'invention. L'invention a aussi pour objet un produit comprenant une souche ou un variant de ladite souche selon l'invention ou un mélange de microorganismes selon l'invention, caractérisé en ce que ledit produit est sous la forme de poudre lyophilisée, de comprimé comprenant ladite poudre lyophilisée et éventuellement des nutriments, ou sous la forme d'une solution aqueuse.

Il est également décrit une enzyme capable de dégrader l'acide polylactique, caractérisée en ce qu'elle est produite par une souche ou un variant de ladite souche selon l'invention.

L'invention concerne également l'utilisation d'une souche ou d'un variant de ladite souche selon l'invention, ou de ladite enzyme selon l'invention, pour dégrader l'acide polylactique.

L'invention concerne aussi un procédé de dégradation de l'acide polylactique, caractérisé en ce qu'il comprend une étape consistant à mettre en contact de l'acide polylactique devant être dégradé avec une souche ou un variant de ladite souche selon l'invention, ou avec une enzyme capable de dégrader l'acide polylactique selon l'invention.

### DEFINITIONS

Selon l'invention, on entend par « variant »:
- un variant naturel d'une souche selon l'invention, c'est à dire un variant obtenu spontanément à partir d'une souche selon l'invention après incubation dans un milieu de sélection. Un variant naturel est donc obtenu sans aucune manipulation génétique de l'opérateur mais seulement par mutation naturelle de la souche et sélection de cette souche mutée dans un milieu approprié, ou
- un variant d'une souche selon l'invention comprenant au moins une mutation dans son génome, ladite mutation étant induite par génie-génétique, par exemple par mutagénèse dirigée ou mutagénèse aléatoire. Par exemple, la mutagénèse aléatoire peut être effectuée à l'aide de mutagènes tels que les radiations (rayons UV, radiations ionisantes, chaleur) ou des composés chimiques (acide nitreux, éthyl-méthanesulfonate, N-méthyl-N'-nitro-N-nitrosoguanine, N-éthyl-N-nitrosourea, acridine orange, proflavine, etc.).

Par « mutation », on entend l'addition, la délétion ou la substitution d'au moins un nucléotide dans le génome de la souche selon l'invention.

Selon l'invention, on entend par milieu CE ou extrait de compost un milieu de culture des bactéries et de leurs variants selon l'invention préparé selon le procédé suivant :
Procédé de préparation pour 1 L de milieu CE :
   100 g de compost sont infusés dans 1 L qsp d'eau ultrapure (Système MilliQ de Millipore), sous agitation durant 16 h (pour la nuit). Le mélange est ensuite centrifugé durant 1 h à 4000 g, avant d'être soumis à une filtration clarifiante, réalisée à l'aide d'un filtre de cellulose (Whatman, Grade 4, pores 20-25 m).
   Pour obtention d'un milieu CE solide (ou « gélosé »), de l'agar peut être ajouté à hauteur de 1,5 % (m/v).

Le milieu est ensuite autoclavé durant 20 min à 120 °C (1 bar).

Par milieu minéral minimum selon l'invention on entend un milieu de culture des bactéries et de leurs variants selon l'invention comprenant :
- 10 mg/L FeSO₄ 7H₂O,
- 200 mg/L MgSO₄ 7H₂O,
- 1 g/L (NH₄)2SO₄,
- 20 mg/L CaCl₂ 2H₂O,
- 100 mg/L NaCl,
- 0,5 mg/L Na₂MoO₄ 2H₂O,
- 0,5 mg/L Na₂WO₄,
- 0,5 mg/L MnSO₄,
- 100 mg/L d'extrait de levure,
- 10,7 mM Tris-HCl,
- pH 8
- Eau ultrapure (Système MilliQ de Millipore), qsp.

Le milieu est ensuite autoclavé durant 20 min à 120 °C (1 bar).

Par milieu ISP2 selon l'invention on entend un milieu de culture des bactéries et de leurs variants selon l'invention comprenant
- extrait de levure 4 g/L,
- extrait de malt 10 g/L,
- glucose 4 g/L)
- Eau ultrapure (Système MilliQ de Millipore), qsp.

Le milieu est ensuite autoclavé durant 20 min à 120 °C (1 bar).

### DESCRIPTION DÉTAILLÉE DE L'INVENTION

Il est décrit une souche de bactérie du genre *Ochrobactrum,* caractérisée en ce que ladite souche est capable de dégrader l'acide polylactique.

L'invention a pour objet une souche de bactérie du genre *Ochrobactrum* déposée selon la Traité de Budapest le 23 juillet 2009 au nom du Centre National de la Recherche Scientifique (3 rue Michel Ange, 75794 Paris Cedex 16) à la Collection Nationale de Cultures de Microorganismes (CNCM, Institut Pasteur, 25 rue du docteur Roux, F-75724 Paris Cedex 15) sous le numéro CNCM I-4212, ou un variant de ladite souche, ledit variant étant capable de dégrader l'acide polylactique, et ledit variant étant un variant naturel de ladite souche ou un variant de ladite souche comprenant au moins une mutation dans son génome.

Les inventeurs ont en effet réussi à sélectionner et caractériser une souche bactérienne à partir d'un compost de jardin, capable de dégrader l'acide polylactique (PLA). Cette souche a pu être affiliée au genre *Ochrobactrum* et a été en conséquence dénommée *Ochrobactrum sp*. 37S (déposée sous le numéro CNCM I-4212).

Le genre *Ochrobactrum* a déjà été isolé pour diverses propriétés de bioremédiation d'environnements diversement pollués : des souches sont capables de dégrader des solvants comme le méthyl-*tert*-butyl-éther, des composés aromatiques, notamment certains dérivés phénoliques comme les nonylphénols ou le 2,4,6-tribromophénol, des organochlorés comme le pesticide endosulfan... Par ailleurs, certains isolats ont montré des capacités à séquestrer les métaux lourds et à réduire différentes formes de chromates et dichromates, à dégrader le chlorure de vinyle (monomère à la base du plastique PVC) ou encore à produire des exopolysaccharides émulsifiants permettant d'augmenter la biodégradation d'hydrocarbures. Thoma et al décrivent un nombre conséquent de différentes bactéries appartenant au genre *Ochrobactrum* (Thoma et al., International Journal of Medical Microbiology, 2009). Toutefois, aucune indication quant à la possibilité pour l'une quelconque de ces souches de dégrader l'acide polylactique est données.A la connaissance des inventeurs, aucune étude ne décrit de souches d'*Ochrobactrum* capable de dégrader le PLA.

Un avantage important des souches du genre *Ochrobactrum* selon l'invention est qu'elles sont capables de dégrader des polymères de PLA de haut poids moléculaire (typiquement compris entre 110 000 et 120 000 Da) dans des milieux simples, en conditions solides comme en conditions liquides. Par ailleurs, les inventeurs ont pu démontrer que la souche *Ochrobactrum sp*. 37S (CNCM I-4212) présente la propriété de dégrader le PLA lorsqu'elle est cultivée sur un milieu CE (extrait de compost), qui est élaboré par simple infusion de compost dans de l'eau. L'ensemble de ces observations montre donc que les souches du genre *Ochrobactrum* selon l'invention, et en particulier la souche *Ochrobactrum sp.* 37S (CNCM I-4212) et ses variants, sont d'excellents candidats pour de nombreuses applications visant à améliorer la dégradation du PLA, en particulier en conditions de compost industriel.

La capacité des souches et de leurs variants selon l'invention à dégrader l'acide polylactique peut être évaluée par toute méthode connue par l'homme du métier. En particulier, la capacité des souches et de leurs variants selon l'invention à dégrader l'acide polylactique peut être typiquement évaluée à l'aide d'un Test A comprenant les étapes suivantes:
1) ensemencer par la méthode des stries une boite de Petri comprenant un milieu minéral minimum gélosé additionné d'acide polylactique émulsionné à 1g/L, à partir d'une culture liquide dans le même milieu comprenant 10⁸ à 10⁹ UFC/mL, de ladite souche ou dudit variant dont on souhaite évaluer la capacité à dégrader l'acide polylactique,
2) incuber la boite de Petri ensemencée à l'étape (1) dans une étuve humide (86-88 % d'humidité) pendant 22 jours à 37°C, et
3) détecter l'éventuelle présence d'une zone translucide autour des colonies ayant poussé sur la boite de Petri, ladite zone translucide révélant la capacité de ladite souche ou dudit variant testé(e) à dégrader l'acide polylactique.

La «méthode des stries» est bien connue de l'homme du métier. Elle consiste typiquement à ensemencer la boite de Petri à l'aide d'une anse de platine préalablement plongée dans une culture liquide de bactéries, en réalisant des stries dans un mouvement de va-et-vient en surface du milieu gélosé.

Pour obtenir le milieu minéral minimum additionné d'acide polylactique tel qu'utilisé à l'étape 1 du Test A selon l'invention on émulsionne dans un milieu minéral minimum selon l'invention 1g/L d'acide polylactique de poids moléculaire compris entre 110 000 et 120 000 Da. L'émulsion est réalisée de la façon suivante : Le PLA sous forme de poudre (polymères de poids moléculaire compris entre 110 000 et 120 000 Da, VALAGRO, Poitiers, France), est tout d'abord solubilisé dans du dichlorométhane (50 g/L de dichlorométhane), avant d'être additionné au milieu d'intérêt à la concentration finale de 1g/L. Le mélange est ensuite homogénéisé par dispersion du PLA dans le milieu à l'aide d'un Ultraturax®, à vitesse maximale, durant 30 à 40 s. Le milieu est alors immédiatement autoclavé (20 min, 120°C, 1 bar), pour éviter la formation d'aggrégats.

Enfin, pour obtenir un milieu gélosé, on ajoute audit milieu minéral minimum additionné d'acide polylactique 1,5% (m/v) d'agar. Ce milieu minéral minimum gélosé additionné d'acide polylactique émulsionné à 1g/L est alors coulé dans des boites de Petri.

L'invention concerne également un mélange de microorganismes caractérisé en ce qu'il comprend une souche ou un variant de ladite souche selon l'invention. En particulier, ce mélange de microorganismes peut comprendre d'autres microorganismes possédant la propriété de dégrader l'acide polylactique. Typiquement, ces microorganismes sont des bactéries ou des champignons. Plus particulièrement, ces microorganismes peuvent être choisis parmi les bactéries appartenant au phylum des Firmicutes (*Firmus cutis*), présentant la propriété de dégrader les polymères de PLA de faible poids moléculaire (inférieur ou égal à 20 000 Da), comme décrit par Mayumi et al. (Mayumi, D. et al. (2008), Applied Microbiology and Biotechnology 79, 743-750). D'autres exemples de bactéries sont les bactéries de la classe des bacilles, notamment les souches de bactéries *Bacillus cereus spp*. ou les souches de bactéries *Bacillus clausii spp*.

Les souches et leurs variants selon l'invention ainsi que les mélanges de microorganismes selon l'invention sont typiquement préparés sous des formes variées. L'invention concerne donc également un produit (ou une composition) comprenant une souche ou un variant de ladite souche selon l'invention ou un mélange de microorganismes selon l'invention, caractérisé en ce que ledit produit (ou ladite composition) est sous la forme de poudre lyophilisée, de comprimé comprenant ladite poudre lyophilisée et éventuellement des nutriments (tels que des vitamines, des sels minéraux, etc....), ou sous la forme d'une solution aqueuse. Typiquement, ladite solution aqueuse est obtenue en cultivant au moins une souche ou un de ses variants selon l'invention ou éventuellement un mélange de microorganismes selon l'invention dans un milieu de culture approprié, tel que le milieu minéral minimum selon l'invention ou le milieu CE (extrait de compost) selon l'invention.

Il est également décrit une enzyme capable de dégrader l'acide polylactique, caractérisée en ce qu'elle est produite par une souche ou un variant de ladite souche selon l'invention. Typiquement, cette enzyme, ou PLA dépolymérase, peut-être carctérisée par clonage du gène codant pour ladite enzyme. Cette caractérisation de l'enzyme par clonage peut notamment être réalisée en suivant un procédé adapté de celui décrit par Mayumi, D. et al., 2008, Applied Microbiology and Biotechnology 79, 743-750. Typiquement, la caractérisation de l'enzyme par clonage du gène codant pour ladite enzyme comprend les étapes suivantes:
1) l'ADN purifié à partir d'une culture de la souche *Ochrobactrum sp*. 37S (déposée sous le numéro CNCM I-4212) cultivée en milieu complet ISP2 est extrait puis digéré de manière partielle à l'aide de l'enzyme *Sau3*AI ;
2) les fragments obtenus compris entre 2 et 4 kb sont ensuite clonés dans le plasmide pUC18 (Toyobo, Osaka, Japon), préalablement digéré avec l'enzyme *Bam*HI ;
3) les plasmides recombinants sont insérés par transformation dans la souche receveuse d'*E*. *Coli* DH5α ;
4) 40 000 clones de bactéries *E. Coli* DH5α transformées (couvrant statistiquement l'intégralité du génome de la souche *Ochrobactrum sp*. 37S) sont étalés, à raison de 500 clones par boite environ, sur des boites de Petri comprenant un milieu LB (Luria-Bartani) gélosé recouvert d'un milieu comprenant 1g/L de PLA émulsionné (poids moléculaire compris entre 110 000 et 120 000 Da) et de 1,5 % d'agar (milieu adapté de Akutsu-Shigeno Y. et al., 2003, vol. 69, 2498-2504),
5) les plasmides sont extraits des bactéries formant une zone translucide sur les boites de Petri;
6) le gène codant pour l'enzyme (PLA dépolymérase) est finalement identifié par séquençage de l'insert et comparaison avec les séquences d'enzymes connues (BLAST).

Afin de faciliter les étapes ultérieures de purification de l'enzyme, une étiquette correspondant à un motif His•Tag® est typiquement introduite, après clonage du gène d'intérêt dans un plasmide d'expression bactérien du type pET-24a(+) (Novagen, Madison, USA). L'enzyme peut alors être purifiée en une étape, par passage d'un extrait enzymatique brut (obtenu après sonication de la souche bactérienne transformée) sur une colonne de nickel (Pharmacia, Biotech, Upsala, Suède). L'activité PLA dépolymérase dans les fractions éluées de la colonne est alors déterminée en mesurant la diminution de turbidité d'une émulsion de PLA, comme décrit par Teeraphatpornchai et al. (2003, Biotechnology Letters, 25, 23-28.). Les fractions présentant l'activité PLA dépolymérase sont alors poolées, puis concentrées par ultracentrifugation (membranes YM10 ; Millipore, Bedford, USA).

L'invention concerne également l'utilisation d'une souche ou d'un variant de ladite souche selon l'invention, ou d'une enzyme selon l'invention, pour dégrader l'acide polylactique.

L'invention concerne aussi un procédé de dégradation de l'acide polylactique, caractérisé en ce qu'il comprend une étape consistant à mettre en contact de l'acide polylactique devant être dégradé avec une souche ou un variant de ladite souche selon l'invention, ou avec une enzyme capable de dégrader l'acide polylactique selon l'invention.

Dans un mode de réalisation particulier, ladite étape consistant à mettre en contact l'acide polylactique devant être dégradé avec une souche ou un variant de ladite souche selon l'invention consiste à ensemencer ledit acide polylactique devant être dégradé avec une souche ou un variant de ladite souche selon l'invention.

Le procédé selon l'invention est donc particulièrement bien adapaté pour dégrader l'acide polylactique dans les milieux comprenant de l'acide polylactique ou dans les milieux comprenant un matériau constitué par tout au partie d'acide polylactique, comme typiquement un mélange de déchets domestiques ou industriels, un mélange de déchets organiques, notamment un compost, un lisier, des boues activées, etc.

Le procédé de dégradation de l'acide polylactique selon l'invention trouve donc de multiples applications, notamment pour traiter les déchets comprenant de l'acide polylactique. Par ailleurs, le procédé de dégradation de l'acide polylactique selon l'invention possède l'avantage de ne pas produire de gaz à effet de serre, à la différence des procédés classiques d'incinération. Par ailleurs, un avantage majeur du procédé selon l'invention est la conversion par les bactéries et leurs variants selon l'invention ou par l'enzyme selon l'invention du PLA en substance organique valorisable, notamment en acides organiques.

Typiquement, l'acide polylactique ou PLA dégradé par les souches de bactéries ou leurs variants selon l'invention est choisi parmi:
- un homopolymère d'acide lactique de conformation L,
- un homopolymère d'acide lactique de conformation D,
- un copolymère d'acide lactique de conformation L et d'acide lactique de conformation D, ou
- un copolymère d'au moins l'un quelconque des polymères mentionnés précédemment avec un autre polymère, l'acide lactique représentant en poids au moins 90% de la composition dudit copolymère.

Toujours typiquement, l'acide polylactique ou PLA dégradé par les souches de bactéries ou leurs variants selon l'invention comprend également un matériau constitué par tout ou partie d'acide polylactique tel que défini précédemment. Selon l'invention, un matériau constitué en partie d'acide polylactique est typiquement un matériau constitué en partie par de l'acide polylactique tel que défini précédemment et en partie par au moins un autre constituant, tel que notamment au moins un autre bioplastique (écopolymère) ou un plastique non biodégradable.

Dans un mode de réalisation particulier, ledit acide polylactique dégradé par les souches de bactéries ou leurs variants selon l'invention est un acide polylactique dont le poids moléculaire est inférieur à 140 000 Da, en particulier compris entre 2 000 et 140 000 Da. De manière tout à fait particulière, ledit acide polylactique dégradé par les souches de bactéries ou leurs variants selon l'invention possède un poids moléculaire compris entre 100 000 et 130 000 Da, encore plus particulièrement entre 110 000 et 120 000 Da.

D'autres aspects et avantages de la présente invention sont décrits dans les exemples suivants, qui doivent être considérés à titre illustratif et comme ne limitant pas la portée de l'invention.

### EXEMPLES

### 1. Isolement de la souche CNCM I-4212:

Une souche présentant la capacité à dégrader le PLA a été isolée à partir d'un compost de jardin comme suit :
- Une plaque de PLA (85 mm X 120 mm X 1 mm ; poids moléculaire de 110 000 à 120 000 Da, VALAGRO, Poitiers, France) a été incubée dans un simple compost de jardin (Iteuil, département de la Vienne, France), durant 45 jours. Le matériel biologique s'étant développé à la surface de la plaque de PLA (biofilm) a ensuite été récupéré par grattage à l'aide d'une spatule métallique ;
- 100 mg de biofilm ont ensuite été resuspendus dans 1 mL de Tampon Phosphate (0,1 M, pH 7). 250 µL de cette suspension ont alors été utilisés pour inoculer un milieu CE liquide contenant une émulsion de PLA (polymères de poids moléculaire compris entre 110 000 et 120 000 Da) à 1 g/L, préparé comme décrit au paragraphe 3.2.2 ci après (« milieu liquide CE + PLA »). La préculture ainsi obtenue a été incubée durant 3 jours à 37°C, sous agitation (200 rpm) ;
- Une étape d'enrichissement (multiplication) de la souche de bactérie dans ledit milieu liquide CE + PLA a ensuite été réalisée par deux ré-inoculations successives (dilutions 10⁻¹) dans 5 mL du même milieu, et dans les mêmes conditions (3 jours d'incubation à 37°C et sous agitation) ;
- Enfin, une aliquote de la dernière culture a été prélevée pour obtenir une suspension dans l'eau milliQ stérile, à la dilution de 10⁻⁵. 100 L de cette dilution ont ensuite été étalés sur un milieu CE gélosé contenant une émulsion de PLA (polymères de poids moléculaire compris entre 110 000 et 120 000 Da), à 1 g/L, préparé comme décrit au paragraphe 3.1.2 ci après. Les boites ont alors été incubées dans une étuve pendant 22 jours à 37°C.

Sur ce milieu, nous avons isolé une colonie entourée par une zone translucide, témoignant de la dégradation du PLA. Cette souche a été récupérée, re-suspendue et étalée par la méthode des stries sur le même milieu.

### 2. Identification de la souche CNCM I-4212:

La croissance de la souche isolée (cf. §1.) a tout d'abord été testée dans différents milieux complets liquides. Nous avons pu montrer que cette souche croît fortement dans un milieu ISP2 (Extrait de levure 4 g/L, extrait de malt 10 g/L, glucose 4 g/L, eau purifiée qsp). A partir d'une culture liquide dans ce milieu, l'ADN total a été extrait comme décrit par Schäfer et Muyzer (Schäfer, H., and Muyzer, G. (2001) Methods in Microbiology, vol. 30, ed. J.H. Paul, London: Academic Press, 425-468). Une portion du gène codant l'ARN 16S a alors été amplifiée à l'aide d'amorces spécifiques (SEQ ID NO :1 et SEQ ID NO :2) et séquencée.

Les séquences des amorces utilisées pour la caractérisation sont les suivantes (Muyzer G. et al. (1993), Appl. Environ. Microbiol. 59, 695-700) : 
341F-GC : (SEQ ID NO :1)
5'CGCCCGCCGCGCCCCGCGCCCGTCCCGCCGCCCCCGCCCGCCTACGGG
AGGCAGCAG3'
 et
907R : (SEQ ID NO :2)
^{5'}CCGTCAATTCCTTTRAGTTT^{3'}

La comparaison de la séquence obtenue avec les banques de données (Blast, *ch.embnet.org*) a permis d'affilier, après réalisation d'un arbre phylogénétique, la souche au genre *Ochrobactrum*. La souche CNCM I-4212 a donc été désignée par les inventeurs sous le nom suivant : *Ochrobactrum sp*. 37S.

### 3. Dégradation de l'acide polylactique :

### 3.1. En milieu gélosé :

La capacité de la souche CNCM I-4212 à dégrader de l'acide polylactique de poids moléculaire compris entre 110 000 et 120 000 Da a été testée sur différents milieux gélosés additionnés de PLA.

### 3.1.1. Milieu minéral minimum gélosé additionné d'acide polylactique de poids moléculaire compris entre 110 000 et 120 000 Da :

Pour obtenir ledit milieu minéral minimum additionné d'acide polylactique on a émulsionné dans un milieu minéral minimum selon l'invention 1g/L d'acide polylactique de poids moléculaire compris entre 110 000 et 120 000 Da.

L'émulsion est réalisée de la façon suivante :
Le PLA sous forme de poudre (polymères de poids moléculaire compris entre 110 000 et 120 000 Da, VALAGRO, Poitiers, France), est tout d'abord solubilisé dans du dichlorométhane (50 g/L de dichlorométhane), avant d'être additionné au milieu d'intérêt à la concentration finale de 1g/L. Le mélange est ensuite homogénéisé par dispersion du PLA dans le milieu à l'aide d'un Ultraturax®, à vitesse maximale, durant 30 à 40 s. Le milieu est alors immédiatement autoclavé (20 min, 120°C, 1 bar), pour éviter la formation d'aggrégats. Enfin, pour obtenir un milieu gélosé on ajoute audit milieu minéral minimum additionné d'acide polylactique, 1,5% (m/v) d'agar. Ce milieu minéral minimum gélosé additionné d'acide polylactique émulsionné à 1g/L est alors coulé dans des boites de Petri.

### 3.1.2. Milieu CE gélosé additionné d'acide polylactique de poids moléculaire compris entre 110 000 et 120 000 Da :

Pour obtenir ledit milieu CE additionné d'acide polylactique on a émulsionné dans un milieu CE selon l'invention 1g/L d'acide polylactique de poids moléculaire compris entre 110 000 et 120 000 Da, comme décrit au paragraphe 3.1.1. Pour obtenir un milieu gélosé on ajoute audit milieu CE additionné d'acide polylactique 1,5% (m/v) d'agar, puis on le coule dans des boites de Petri.

### 3.1.3. Ensemencement

Des boites de Petri préparées comme décrit aux paragraphes 3.1.1. et 3.1.2. ont été ensemencées par la méthode des stries à partir d'une culture liquide (milieu CE ou milieu minéral minimum) comprenant 10⁸ à 10⁹ UFC/mL de la souche CNCM I-4212. Les boites ont alors été incubées dans une étuve pendant 22 jours à 37°C.

### 3.1.4. Observations

La dégradation du PLA a été évaluée au cours du temps (de 5 à 22 jours après l'ensemencement) par apparition d'une zone translucide claire autour des colonies de la souche de bactérie selon l'invention.

Après 22 jours d'incubation, quelque soit le milieu de culture utilisé, les colonies de la souche de bactérie selon l'invention étaient entourées d'une zone claire translucide témoignant de la dégradation du PLA.

Cependant, la culture sur un milieu minéral minimum gélosé additionné d'acide polylactique permet d'accélérer la dégradation du PLA par rapport à celle observée sur un milieu CE gélosé additionné d'acide polylactique.

### 3.2. En milieu liquide :

La capacité de la souche CNCM I-4212 à dégrader de l'acide polylactique de poids moléculaire compris entre 110 000 et 120 000 Da a également été évaluée en milieu liquide.

### 3.2.1. Milieu minéral minimum liquide additionné d'acide polylactique de poids moléculaire compris entre 110 000 et 120 000 Da :

Pour obtenir le milieu minéral minimum liquide additionné d'acide polylactique on a émulsionné dans un milieu minéral minimum selon l'invention 1g/L d'acide polylactique de poids moléculaire compris entre 110 000 et 120 000 Da.

L'émulsion a été réalisée comme décrit au paragrpahe 3.1.1.

### 3.2.2. Milieu CE liquide additionné d'acide polylactique de poids moléculaire compris entre 110 000 et 120 000 Da :

Pour obtenir le milieu CE liquide additionné d'acide polylactique on a émulsionné dans un milieu CE selon l'invention 1g/L d'acide polylactique de poids moléculaire compris entre 110 000 et 120 000 Da, comme décrit au paragraphe 3.1.1.

### 3.2.3. Inoculation

200 mL de milieu CE ou de milieu minéral minimum préparés comme décrit aux paragraphes 3.2.1 et 3.2.2 ont été inoculés, ou non (témoins), avec environ 5.10⁹ UFC de la souche CNCM I-4212, puis incubés pendant 30 jours à 37°C sous agitation à 150 rpm.

### 3.2.4. Observations

Une fois l'incubation terminée (J+30), le PLA a été extrait des milieux de culture avec du dichlorométhane. Plus spécifiquement, 200 mL de dichlorométhane ont été additionnés aux 200 mL de culture. Après obtention d'une phase homogène par agitation vigoureuse, le mélange est mis à décanter durant 10 min, avant prélèvement de la phase supérieure et son transfert dans un bécher en verre de 1 L. Après évaporation complète du solvant (3 jours sous hotte aspirante (Sorbonne)), le PLA restant (non dégradé) est observé par apparition d'un dépôt brun dans le bécher. Les résultats ont montré que le dépôt de PLA restant est significativement diminué en présence de la souche CNCM I-4212. Les mêmes résultats ont été obtenus quelque soit le milieu de culture.

Ces résultats démontrent que la souche CNCM I-4212 est capable de dégrader des polymères de PLA de poids moléculaire élevé en conditions liquides, et qui plus est dans un milieu simple composé d'un extrait de compost, le milieu CE.

## Revendications

1. Souche de bactérie du genre *Ochrobactrum* déposée selon la Traité de Budapest le 23 juillet 2009 au nom du Centre National de la Recherche Scientifique à la Collection Nationale de Cultures de Microorganismes sous le numéro CNCM I-4212, ou un variant de ladite souche,
ledit variant étant capable de dégrader l'acide polylactique,
ledit variant étant un variant naturel de ladite souche ou un variant de ladite souche comprenant au moins une mutation dans son génome.

2. Mélange de microorganismes **caractérisé en ce qu'**il comprend une souche ou un variant de ladite souche selon la revendication 1.

3. Produit comprenant une souche ou un variant de ladite souche selon la revendication 1 ou un mélange de microorganismes selon la revendication 2, **caractérisé en ce que** ledit produit est sous la forme de poudre lyophilisée, de comprimé comprenant ladite poudre lyophilisée et éventuellement des nutriments, ou sous la forme d'une solution aqueuse.

4. Utilisation d'une souche ou d'un variant de ladite souche selon la revendication 1 pour dégrader l'acide polylactique.

5. Utilisation selon la revendication 4, **caractérisée en ce que** ledit acide polylactique possède un poids moléculaire inférieur à 140 000 Da.

6. Procédé de dégradation de l'acide polylactique, **caractérisé en ce qu'**il comprend une étape consistant à mettre en contact de l'acide polylactique devant être dégradée avec une souche ou un variant de ladite souche selon la revendication 1.

7. Procédé selon la revendication 6, **caractérisé en ce que** ledit acide polylactique possède un poids moléculaire inférieur à 140 000 Da.

## Patentansprüche

1. Bakterienstamm der Gattung *Ochrobactrum,* welcher nach dem Budapester Vertrag am 23. Juli 2009 im Namen des Centre National de la Recherche Scientifique an der Collection Nationale de Cultures de Microorganismes unter der Nummer CNCM I-4212 hinterlegt wurde, oder eine Variante des Stamms,
wobei die Variante die Polymilchsäure abbauen kann,
wobei die Variante eine natürliche Variante des Stamms oder eine Variante des Stamms, umfassend mindestens eine Mutation in seinem Genom, ist.

2. Gemisch von Mikroorganismen, **dadurch gekennzeichnet, dass** es einen Stamm oder eine Variante des Stamms nach Anspruch 1 umfasst.

3. Produkt, umfassend einen Stamm oder eine Variante des Stamms nach Anspruch 1 oder ein Gemisch von Mikroorganismen nach Anspruch 2, **dadurch gekennzeichnet, dass** das Produkt in Form eines lyophilisierten Pulvers, einer Tablette umfassend das lyophilisierte Pulver und gegebenenfalls Nährstoffe, oder in Form einer wässrigen Lösung ist.

4. Verwendung eines Stamms oder einer Variante des Stamms nach Anspruch 1 zum Abbauen von Polymilchsäure.

5. Verwendung nach Anspruch 4, **dadurch gekennzeichnet, dass** die Polymilchsäure ein Molekulargewicht von weniger als 140 000 Da aufweist.

6. Verfahren zum Abbauen von Polymilchsäure, **dadurch gekennzeichnet, dass** es einen Schritt aufweist, welcher darin besteht, abzubauende Polymilchsäure mit einem Stamm oder einer Variante des Stamms nach Anspruch 1 in Kontakt zu bringen.

7. Verfahren nach Anspruch 6, **dadurch gekennzeichnet, dass** die Polymilchsäure ein Molekulargewicht von weniger als 140 000 Da aufweist.

## Claims

1. A strain of bacteria of the *Ochrobactrum* genus, deposited according to the Treaty of Budapest on July 23, 2009, in the name of the Centre National de la Recherche Scientifique, at the Collection Nationale de Cultures de Microorganismes under number CNCM I-4212, or a variant of said strain,
said variant being capable of degrading polylactic acid,
said variant being a natural variant of said strain or a variant of said strain comprising at least one mutation in its genome.

2. A microorganism mixture, **characterized in that** it comprises a strain or a variant of said strain as claimed in claim 1 or 2.

3. A product comprising a strain or a variant of said strain as claimed in claim 1 or a microorganism mixture as claimed in claim 2, **characterized in that** said product is in the form of a freeze-dried powder, of a tablet comprising said freeze-dried powder and, optionally, nutrients, or in the form of an aqueous solution.

4. The use of a strain or of a variant of said strain as claimed in claim 1 for degrading polylactic acid.

5. The use as claimed in claim 4, **characterized in that** said polylactic acid has a molecular weight of less than 140 000 Da.

6. A process for degrading polylactic acid, **characterized in that** it comprises a step consisting in bringing polylactic acid that has to be degraded into contact with a strain or of a variant of said strain as claimed in claim 1.

7. The process as claimed in claim 6, **characterized in that** said polylactic acid has a molecular weight of less than 140 000 Da.
